# EUROPEAN PATENT APPLICATION

(11) **EP 3 607 957 A1**
(43) Date of publication of application: **12.02.2020**
(21) Application number: 18794056.4
(22) Date of filing: 25.04.2018
(51) Int. Cl.: A61K 35/12, A61K 35/545, A61P 17/00, A61P 17/02, A61P 17/14, A61P 43/00

(54) **PHARMACEUTICAL COMPOSITION AND COSMETIC COMPOSITION**

(30) Priority: 02.05.2017 US 201762500117 P
(71) Applicant: Tanabe, Koji, Palo Alto, CA 94303 (US); I Peace, Inc., Palo Alto, CA 94303 (US)
(72) Inventor: TANABE, Koji, Palo Alto, California 94303 (US); DATE, Akira, Kyoto-shi Kyoto 606-8414 (JP); SUTO, Kenta, Palo Alto, California 94303 (US)
(74) Representative: MacLean, Martin Robert
(86) International application number: PCT/JP2018/016738
(87) International publication number: WO 2018/203499

(57) **Abstract**

Provided is a pharmaceutical composition including a supernatant of a culture medium that has been used for maintenance culture of stem cells in an undifferentiated state. The stem cells for the pharmaceutical composition may be pluripotent stem cells.

## Description

### FIELD

The present invention relates to a pharmaceutical composition and to a cosmetic composition.

### BACKGROUND

Embryonic stem cells (ES cells) are stem cells established from early embryos of humans or mice, for example. ES cells are pluripotent, being capable of differentiating into all cells in the body. At the current time, human ES cells are able to be used in cell transplantation therapy for numerous diseases including Parkinson's disease, juvenile onset diabetes and leukemia. However, certain barriers exist against transplantation of ES cells. In particular, transplantation of ES cells can provoke immunorejection similar to the rejection encountered after unsuccessful organ transplantation. Moreover, there are many ethical considerations as well as critical and dissenting opinions against the use of ES cell lines that have been established by destruction of human embryos.

It was against this background that Professor Shinya Yamanaka of Kyoto University successfully established a line of induced pluripotent stem cells (iPS cells) by transferring four genes: Oct3/4, Klf4, c-Myc and Sox2, into somatic cells. For this, Professor Yamanaka received the Nobel Prize in Physiology or Medicine in 2012 (see PTL 1, for example). iPS cells are ideal pluripotent cells which are free of issues of rejection or ethical problems. Therefore, iPS cells are considered promising for use in cell transplantation therapy. It has been reported that culture media used for culturing of iPS cells have been reutilized in pharmaceutical compositions (see PTL 2, for example).

### [CITATION LIST]

### [PATENT LITERATURE]

PTL 1 : Japanese Patent Publication No. 4183742
PTL 2 : Japanese Unexamined Patent Publication No. 2016-128396

### SUMMARY

### [TECHNICAL PROBLEM]

As far as has been verified by the present inventors, however, the iPS cells in the culturing method described in PTL 2 become differentiated, and therefore presumably the medium that is reutilized is that obtained from culturing of not iPS cells, but rather differentiated cells. It is an object of the present invention to provide a pharmaceutical composition and a cosmetic composition that effectively use culturing media of stem cells such as iPS cells.

### [SOLUTION TO PROBLEM]

As a result of conducting much diligent research, the present inventors have found that a supernatant of culture medium that has been used for maintenance culture of stem cells in an undifferentiated state can serve as an active ingredient in a pharmaceutical or cosmetic.

According to one aspect of the invention there is provided a pharmaceutical composition or a pharmaceutical composition starting material that includes a supernatant of a culture medium that has been used for maintenance culture of stem cells in an undifferentiated state. In other words, there is provided a supernatant of a culture medium that has been used for maintenance culture of stem cells in an undifferentiated state, to be used in a pharmaceutical composition or a pharmaceutical composition starting material. The stem cells in the pharmaceutical composition or pharmaceutical composition starting material may be pluripotent stem cells. The pluripotent stem cells may be iPS cells. The pluripotent stem cells may also be ES cells. The culture medium may be a gel medium. The culture medium may also include gellan gum.

According to one aspect of the invention there is provided a treatment method that includes administration or application of a pharmaceutical composition that includes a supernatant of a culture medium that has been used for maintenance culture of stem cells in an undifferentiated state, to a subject. The subject may be a human or a non-human animal, or a plant.

According to one aspect of the invention there is provided an agent that prevents formation of or ameliorates skin blemishes, wrinkles or sagging, which comprises the aforementioned pharmaceutical composition. According to another aspect of the invention there is provided a treatment method that includes administering or applying to a subject, an agent that prevents formation of or ameliorates skin blemishes, wrinkles or sagging, which comprises the aforementioned pharmaceutical composition. The subject may be a human or a non-human animal.

According to another aspect of the invention there is provided a cosmetic composition or a cosmetic composition starting material that includes a supernatant of a culture medium that has been used for maintenance culture of stem cells in an undifferentiated state. In other words, there is provided a supernatant of a culture medium that has been used for maintenance culture of stem cells in an undifferentiated state, to be used in a cosmetic or a cosmetic composition starting material. The stem cells in the cosmetic composition or cosmetic composition starting material may be pluripotent stem cells. The pluripotent stem cells may be iPS cells. The pluripotent stem cells may also be ES cells. The culture medium may be a gel medium. The culture medium may also include gellan gum.

According to one aspect of the invention there is provided an agent that prevents formation of or ameliorates skin blemishes, wrinkles or sagging, which comprises the aforementioned cosmetic composition. According to another aspect of the invention there is provided a treatment method that includes administering or applying to a subject, an agent that prevents formation of or ameliorates skin blemishes, wrinkles or sagging, which comprises the aforementioned cosmetic composition. The subject may be a human or a non-human animal.

According to another aspect of the invention there is provided a collagen production promoter or a collagen production promoter starting material, that includes a supernatant of a culture medium that has been used for maintenance culture of stem cells in an undifferentiated state. In other words, there is provided a supernatant of a culture medium that has been used for maintenance culture of stem cells in an undifferentiated state, to be used for promoting collagen production. The stem cells in the collagen production promoter or collagen production promoter starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a treatment method that includes administration or application of a collagen production promoter that includes a supernatant of a culture medium that has been used for maintenance culture of stem cells in an undifferentiated state, to a subject. The subject may be a human or a non-human animal.

According to another aspect of the invention there is provided a hyaluronic acid production promoter or a hyaluronic acid production promoter starting material, that includes a supernatant of a culture medium that has been used for maintenance culture of stem cells in an undifferentiated state. In other words, there is provided a supernatant of a culture medium that has been used for maintenance culture of stem cells in an undifferentiated state, to be used for promoting hyaluronic acid production. The stem cells in the hyaluronic acid production promoter or hyaluronic acid production promoter starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a treatment method that includes administration or application of a hyaluronic acid production promoter that includes a supernatant of a culture medium that has been used for maintenance culture of stem cells in an undifferentiated state, to a subject. The subject may be a human or a non-human animal.

According to another aspect of the invention there is provided a wound treatment agent or a wound treatment agent starting material that includes a supernatant of a culture medium that has been used for maintenance culture of stem cells in an undifferentiated state. In other words, there is provided a supernatant of a culture medium that has been used for maintenance culture of stem cells in an undifferentiated state, to be used for treatment of wounds. The stem cells in the wound treatment agent or wound treatment agent starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a treatment method that includes administration or application of a wound treatment agent that includes a supernatant of a culture medium that has been used for maintenance culture of stem cells in an undifferentiated state, to a subject. The subject may be a human or a non-human animal.

According to another aspect of the invention there is provided an epidermal cell growth promoter or an epidermal cell growth promoter starting material that includes a supernatant of a culture medium that has been used for maintenance culture of stem cells in an undifferentiated state. In other words, there is provided a supernatant of a culture medium that has been used for maintenance culture of stem cells in an undifferentiated state, to be used for promoting growth of epidermal cells. The stem cells in the epidermal cell growth promoter or epidermal cell growth promoter starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a treatment method that includes administration or application of an epidermal cell growth promoter that includes a supernatant of a culture medium that has been used for maintenance culture of stem cells in an undifferentiated state, to a subject. The subject may be a human or a non-human animal.

According to another aspect of the invention there is provided a hair growth agent or a hair growth agent starting material that includes a supernatant of a culture medium that has been used for maintenance culture of stem cells in an undifferentiated state. In other words, there is provided a supernatant of a culture medium that has been used for maintenance culture of stem cells in an undifferentiated state, to be used for hair growth. The stem cells in the hair growth agent or hair growth agent starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a treatment method that includes administration or application of a hair growth agent that includes a supernatant of a culture medium that has been used for maintenance culture of stem cells in an undifferentiated state, to a subject. The subject may be a human or a non-human animal.

According to another aspect of the invention there is provided a dermal papilla cell activating agent or a dermal papilla cell activating agent starting material that includes a supernatant of a culture medium that has been used for maintenance culture of stem cells in an undifferentiated state. In other words, there is provided a supernatant of a culture medium that has been used for maintenance culture of stem cells in an undifferentiated state, to be used as a dermal papilla cell activating agent or a dermal papilla cell activating agent starting material. The stem cells in the dermal papilla cell activating agent or dermal papilla cell activating agent starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a treatment method that includes administration or application of dermal papilla cells that include a supernatant of a culture medium that has been used for maintenance culture of stem cells in an undifferentiated state, to a subject. The subject may be a human or a non-human animal.

According to another aspect of the invention there is provided a fibroblast growth factor (FGF) family production promoter or an FGF family production promoter starting material that includes a supernatant of a culture medium that has been used for maintenance culture of stem cells in an undifferentiated state. In other words, there is provided a supernatant of a culture medium that has been used for maintenance culture of stem cells in an undifferentiated state, to be used for promoting FGF family production. Examples in the FGF family include FGF-2 and FGF-7. The stem cells in the FGF family production promoter or FGF family production promoter starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a treatment method that includes administration or application of an FGF production promoter that includes a supernatant of a culture medium that has been used for maintenance culture of stem cells in an undifferentiated state, to a subject. The subject may be a human or a non-human animal.

According to another aspect of the invention there is provided a vascular endothelial growth factor (VEGF) production promoter or a VEGF production promoter starting material that includes a supernatant of a culture medium that has been used for maintenance culture of stem cells in an undifferentiated state. In other words, there is provided a supernatant of a culture medium that has been used for maintenance culture of stem cells in an undifferentiated state, to be used for promoting VEGF production. The stem cells in the VEGF production promoter or VEGF production promoter starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a treatment method that includes administration or application of a VEGF production promoter that includes a supernatant of a culture medium that has been used for maintenance culture of stem cells in an undifferentiated state, to a subject. The subject may be a human or a non-human animal.

According to another aspect of the invention there is provided a cytoprotective agent or cytoprotective agent starting material which protects cells from stress, and that includes a supernatant of a culture medium that has been used for maintenance culture of stem cells in an undifferentiated state. In other words, there is provided a supernatant of a culture medium that has been used for maintenance culture of stem cells in an undifferentiated state, to be used for protection of cells from stress. The stem cells in the cytoprotective agent or cytoprotective agent starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a cell viability improver or a cell viability improver starting material which increases the viability of stressed cells, and that includes a supernatant of a culture medium that has been used for maintenance culture of stem cells in an undifferentiated state. In other words, there is provided a supernatant of a culture medium that has been used for maintenance culture of stem cells in an undifferentiated state, to be used to increase the viability of stressed cells. The stem cells in the cell viability improver or cell viability improver starting material may be pluripotent stem cells. The pluripotent stem cells may be iPS cells. The pluripotent stem cells may also be ES cells. The culture medium may be a gel medium. The culture medium may also include gellan gum.

According to another aspect of the invention there is provided a biological substance protective agent or a biological substance protective agent starting material, which protects one or more biological substances selected from the group consisting of nucleic acids, proteins, protein complexes, lipoproteins, ribosomes and biological membranes from stress, and that includes a supernatant of a culture medium that has been used for maintenance culture of stem cells in an undifferentiated state. In other words, there is provided a supernatant of a culture medium that has been used for maintenance culture of stem cells in an undifferentiated state, for protection of one or more biological substances selected from the group consisting of nucleic acids, proteins, protein complexes, lipoproteins, ribosomes and biological membranes from stress. The stem cells in the biological substance protective agent or biological substance protective agent starting material may be pluripotent stem cells. The pluripotent stem cells may be iPS cells. The pluripotent stem cells may also be ES cells. The culture medium may be a gel medium. The culture medium may also include gellan gum.

### [ADVANTAGEOUS EFFECTS OF INVENTION]

According to the invention it is possible to provide a pharmaceutical composition and a cosmetic composition that effectively use stem cell culture media.

### BRIEF DESCRIPTION OF DRAWINGS

Fig. 1 is a graph showing the results of a proliferation test using fibroblasts in Example 4.
Fig. 2 is a graph showing the results of a proliferation test using fibroblasts in Example 4.
Fig. 3 is a graph showing the results of a collagen production test using fibroblasts in Example 5.
Fig. 4 is a graph showing the results of a collagen production test using fibroblasts in Example 5.
Fig. 5 is a graph showing the results of a hyaluronic acid production test using fibroblasts in Example 5.
Fig. 6 is a photograph showing the results of a migration ability test using epidermal cells in Example 6.
Fig. 7 is a graph showing the results of a migration ability test using epidermal cells in Example 6.
Fig. 8 is a graph showing the results of a proliferation test using dermal papilla cells in Example 7.
Fig. 9 is a graph showing the results of an FGF-7 production test using dermal papilla cells in Example 8.
Fig. 10 is a graph showing the results of a VEGF-production test using dermal papilla cells in Example 8.

### DESCRIPTION OF EMBODIMENTS

Embodiments of the invention will now be explained in detail. The embodiments described below are merely examples of devices and methods for implementing the technical concept of the invention, and the technical concept of the invention is not limited to the described combinations of structural members. The technical concept of the invention may incorporate various modifications such as are within the scope of the Claims.

The pharmaceutical composition, pharmaceutical composition starting material, cosmetic composition and cosmetic composition starting material of the embodiments all include a supernatant of a culture medium that has been used for maintenance culture of stem cells in an undifferentiated state. The stem cells are pluripotent stem cells, such as artificial pluripotent stem (iPS) cells or embryonic stem cells (ES cells), for example. The stem cells may be adhesion cultured or suspension cultured.

The stem cell culture medium used may be human ES/iPS culture medium, such as TeSR2 (STEMCELL Technologies), for example. The stem cell culture medium is not limited to this, however, and various stem cell culture media may be used. For example, Primate ES Cell Medium, Reprostem, ReproFF, ReproFF2, ReproXF (Reprocell), mTeSR1, TeSRE8, ReproTeSR (STEMCELL Technologies), PluriSTEM^{R} Human ES/iPS Medium (Merck), NutriStem^{R} XF/FF Culture Medium for Human iPS and ES Cells, Pluriton reprogramming medium (Stemgent), PluriSTEM^{R}, Stemfit AK02N, Stemfit AK03 (Ajinomoto), ESC-Sure^{R} serum and feeder free medium for hESC/iPS (Applied StemCell), L7^{R} hPSC Culture System (LONZA) and Primate ES Cell Medium (ReproCELL) may be used.

Alternatively, the stem cell culture medium may be Dulbecco's Modified Eagle's Medium/Ham's F-12 (DMEM/F12) containing added serum replacement, L-glutamine, non-essential amino acid solution, 2-mercaptoethanol and penicillin/streptomycin. The stem cell culture medium may include growth factors such as basic fibroblast growth factor (bFGF).

Gel medium may be used when the stem cells are to be suspension cultured. The gel medium is prepared, for example, by adding gellan gum such as deacylated gellan gum to the stem cell culture medium, to a final concentration of 0.5 wt% to 0.001 wt%, 0.1 wt% to 0.005 wt% or 0.05 wt% to 0.01 wt%. For the purpose of the present disclosure, gellan gum includes deacylated gellan gum.

The gel medium may include one or more high molecular compounds selected from the group consisting of hyaluronic acid, rhamsan gum, diutan gum, xanthan gum, carrageenan, fucoidan, pectin, pectic acid, pectinic acid, heparan sulfate, heparin, heparitin sulfate, keratosulfate, chondroitin sulfate, dermatan sulfate, rhamnan sulfate, and salts of the foregoing. The gel medium may also include methyl cellulose, and lipids such as lysophosphatidic acid and sphingosine-1-phosphate. Including such substances will help minimize aggregation between the cells.

Alternatively, the gel medium may include at least one temperature-sensitive gel selected from among poly(glycerol monomethacrylate) (PGMA), poly(2-hydroxypropyl methacrylate) (PHPMA), poly(N-isopropylacrylamide) (PNIPAM), amine terminated, carboxylic acid terminated, maleimide terminated, N-hydroxysuccinimide (NHS) ester terminated, triethoxysilane terminated, poly(N-isopropylacrylamide-co-acrylamide), poly(N-isopropylacrylamide-co-acrylic acid), poly(N-isopropylacrylamide-co-butylacrylate), poly(N-isopropylacrylamide-co-methacrylic acid), poly(N-isopropylacrylamide-co-methacrylic acid-co-octadecyl acrylate) and N-isopropylacrylamide.

A ROCK inhibitor may also be added to the gel medium to a final concentration of 1000 µmol/L to 0.1 µmol/L, 100 µmol/L to 1 µmol/L, or 5 µmol/L to 20 µmol/L, for example. Adding a ROCK inhibitor to the gel medium promotes colony formation by the stem cells.

The gel medium does not need to include a growth factor such as bFGF. However, the gel medium may include a growth factor such as bFGF at a low concentration of up to 400 µg/L, up to 100 µg/L, up to 40 µg/L or up to 10 µg/L.

The gel medium may also lack TGF-β, or it may include TGF-β at a low concentration of up to 600 ng/L, up to 300 ng/L or up to 100 ng/L.

Before suspension culturing of the stem cells, they may be dissociated into single cells and the stem cells that have been dissociated into single cells may be placed in the gel medium. The gel medium is not agitated. The single cells are grown while maintaining their clonality and undifferentiated state, forming colonies in the gel medium. It can be confirmed whether or not stem cells have maintained their undifferentiated state, by examining whether or not the cells express an undifferentiated marker.

The temperature during maintenance culture of the stem cells may be 37°C, for example. The carbon dioxide concentration during maintenance culture of the stem cells may be 5%, for example. The period for maintenance culture of the stem cells may be 1 day to 90 days, 2 days to 60 days, 5 days to 30 days or 7 days to 21 days, for example. The supernatant of a culture medium that has been used for maintenance culture of stem cells in an undifferentiated state may have the stem cells removed by filtration, centrifugation or the like.

The pharmaceutical composition of this embodiment may be a skin application composition. The pharmaceutical composition of this embodiment may be a skin disease treatment agent. Examples of diseases that are treatable with the skin disease treatment agent of this embodiment include acne vulgaris, psoriasis vulgaris, keloid, seborrheic dermatitis, contact dermatitis, atopic dermatitis, atopic xeroderma, dermatoporosis, solar elastosis, actinic keratosis, blepharoptosis, alopecia areata, alopecia, eyelash hypotrichosis, liver spots, senile pigmentation, heat rash, freckles, delayed bilateral nevus of Ota, seborrheic keratosis, progeria-related skin diseases, and herpes simplex.

Examples of conditions that can be improved or eliminated by a cosmetic composition of this embodiment include skin spots, freckles, wrinkles, sagging, cracking, reduced skin tightness, loss of skin clarity, sensitive skin, dry skin and thin hair. The effect of the cosmetic composition of this embodiment may be skin improvement, skin texture improvement, healthy maintenance of skin, preventing skin roughening, tightening skin, moisturizing skin, retaining skin moisture and oils, maintaining skin softness, protecting skin, preventing skin dryness, softening skin, providing skin elasticity, providing skin luster, smoothing skin, providing skin elasticity, reducing visibility of skin spots, minimizing wrinkles and lightening skin. Other effects of the cosmetic composition of this embodiment related to the scalp or hair include maintaining healthy scalp, growing hair, preventing hair thinning, preventing itching, preventing alopecia, promoting hair generation, promoting hair growth, preventing post-disease and postpartum alopecia, and restoring hair.

The pharmaceutical composition of this embodiment may be a wound treatment agent, an epidermal cell growth promoter, an epidermal turnover promoter, a hair growth agent, a hair restorer or an eyelash hypotrichosis treatment agent. The pharmaceutical composition or cosmetic composition of this embodiment may be a collagen production promoter, a hyaluronic acid production promoter, a hair growth agent, a fibroblast growth factor (FGF) family production promoter or a vascular endothelial growth factor (VEGF) production promoter.

During hair growth, hair matrix cells of the hair root divide, and the resulting formed cells construct hair. Hair growth takes place in a cycle known as the hair cycle, in which an anagen phase, catagen phase and telogen phase are repeated. Dermal papilla cells affect proliferation and differentiation of follicular epithelial stem cells via production and release of growth factors, thus controlling the hair cycle. Activation of dermal papilla cells and hair matrix cells is said to contribute to the mechanism of hair growth. Active remodeling of blood vessels takes place in the hair follicle during the hair cycle, but if vascularization is impaired during this period, the supply of nutrients and oxygen becomes insufficient for hair formation. Deficient blood flow from the hair follicle vascular network is said to contribute to the pathology of androgenetic alopecia (AGA).

The following is known regarding the connection between dermal papilla cell genes and hair generation and growth. Specifically, FGF-7 and IGF-1 are known to be growth factors secreted by papilla cells to hair matrix cells. These genes function to maintain growth of hair follicles. Vascular endothelial growth factor (VEGF) is secreted by dermal papilla cells and is involved in growth of hair follicle blood vessels, while also having an autocrinic effect of causing proliferation of dermal papilla cells, but its expression level decreases from the anagen phase through to the catagen phase. Expression of the VEGF gene decreases in the hair tissue of AGA (androgenetic alopecia). VEGFB binds competitively to VEGFR-1, the receptor on which VEGF acts. VEGFB exhibits activity that accelerates proliferation and permeability of vascular endothelial cells, but its effect in hair follicles is unknown.

The pharmaceutical composition and cosmetic composition of this embodiment acts directly on the dermal papilla, increasing production of the hair growth acceleration factor FGF-7 to accelerate hair generation, thereby producing an effect of lengthening the anagen phase of the hair cycle and nurturing thin weak hairs to form thicker stronger hairs, while also increasing vascular endothelial growth factor (VEGF) which is secreted by dermal papilla cells and leads to increased hair follicle blood vessels, thus causing proliferation of dermal papilla cells in an autocrine manner.

When the pharmaceutical composition and cosmetic composition of this embodiment are used as a hair restorer or hair growth agent, they may also include other active ingredients such as minoxidil, swertia, pantothenyl ethyl ether, tocopherol acetic acid ester, dipotassium glycyrrhizinate and adenosine.

Examples of wounds that are treatable by a wound treatment agent of this embodiment include burns, abrasion wounds, lacerated wounds, bruises, suture wounds, bedsores and skin defect wounds.

The pharmaceutical composition or cosmetic composition of this embodiment may also be a cytoprotective agent which protects cells from stress, and that includes a supernatant of a culture medium that has been used for maintenance culture of stem cells in an undifferentiated state. The pharmaceutical composition or cosmetic composition of this embodiment may also be a cell viability improver, which stabilizes stressed cells and improves their viability, for example, and that includes a supernatant of a culture medium that has been used for maintenance culture of stem cells in an undifferentiated state. The stressed cells may be fibroblasts, epidermal cells or dermal papilla cells, for example, although they may be any type of cell without limitation to these. Alternatively, the pharmaceutical composition or cosmetic composition of this embodiment may be a biological substance protective agent which protects one or more biological substances selected from the group consisting of nucleic acids, proteins, protein complexes, lipoproteins, ribosomes and biological membranes from stress, and that includes a supernatant of a culture medium that has been used for maintenance culture of stem cells in an undifferentiated state. The biological membranes referred to here include cell membranes.

The pharmaceutical composition and cosmetic composition of this embodiment includes an effective amount of a supernatant of a culture medium that has been used for maintenance culture of stem cells in an undifferentiated state. The effective amount referred to here is an amount capable of exhibiting an effect as a pharmaceutical composition or cosmetic composition. The effective amount is set as appropriate according to the age of the subject, the target condition, the presence or absence of other components and the amounts of other compounds.

The pharmaceutical composition and cosmetic composition of this embodiment may also include pharmaceutically acceptable carriers, such as excipients, disintegrators, buffering agents, emulsifiers, suspending agents, soothing agents, stabilizers, preservatives, antiseptic agents or physiological saline. Examples of excipients include lactose, starch, sorbitol, D-mannitol and saccharose. Examples of disintegrators include carboxymethyl cellulose and calcium carbonate. Examples of buffering agents include phosphates, citrates and acetates. Examples of emulsifiers include gum arabic, sodium alginate and tragacanth.

Examples of suspending agents include glycerin monostearate, aluminum monostearate, methyl cellulose, carboxymethyl cellulose, hydroxymethyl cellulose and sodium lauryl sulfate. Examples of soothing agents include benzyl alcohol, chlorobutanol and sorbitol. Compounds of stabilizers include propylene glycol and ascorbic acid. Examples of preservatives include phenol, benzalkonium chloride, benzyl alcohol, chlorobutanol and methylparaben. Examples of antiseptic agents include benzalkonium chloride, paraoxybenzoic acid and chlorobutanol.

The pharmaceutical composition and cosmetic composition of this embodiment may further include water, an alcohol, a surfactant (cationic, anionic, nonionic or amphoteric surfactant), a humectant (such as glycerin, 1,3-butylene glycol, propylene glycol, propanediol, pentanediol, polyquaternium, an amino acid, urea, a pyrrolidonecarboxylic acid salt, a nucleic acid, a monosaccharide or an oligosaccharide, or any of their derivatives), a thickener (a polysaccharide, polyacrylic acid salt, carboxyvinyl polymer, polyvinylpyrrolidone, polyvinyl alcohol, chitin, chitosan, alginic acid, carrageenan, xanthan gum or methyl cellulose, or any of their derivatives), a wax, vaseline, a hydrocarbon saturated fatty acid, an unsaturated fatty acid or a silicon oil, or any of their derivatives, a triglyceride such as glyceryl tri(caprylate/caprate) or glyceryl trioctanoate, an ester oil such as isopropyl stearate, a natural fat or oil (such as olive oil, camellia oil, avocado oil, almond oil, cocoa butter, evening primrose oil, grape seed oil, macadamia nut oil, eucalyptus oil, rosehip oil, squalane, orange roughy oil, lanolin or ceramide), an antiseptic agent (such as an oxybenzoic acid derivative, dehydroacetic acid salt, light-sensitive element, sorbic acid or phenoxyethanol, or any of their derivatives), a microbicide (sulfur, triclocarbanilide, salicylic acid, zinc pyrithione or hinokitiol, or any of their derivatives), an ultraviolet absorber (paraaminobenzoic acid or methoxycinnamic acid, or any of their derivatives), an anti-inflammatory agent (allantoin, bisabolol, ε-aminocaproic acid, acetyl farnesyl cysteine or glycyrrhizinic acid, or any of their derivatives), an antioxidant (tocopherol, BHA, BHT or astaxanthin, or any of their derivatives), a chelating agent (edetic acid or hydroxyethanediphosphonic acid, or any of their derivatives), an animal or plant extract (ashitaba, aloe, rose fruit, scutellaria root, phellodendron bark, sea weed, Chinese quince, camomile, licorice, kiwi, cucumber, mulberry, white birch, toki (*Angelica acutiloba*)*,* garlic, peony, hop, common horse-chestnut, lavender, rosemary, eucalyptus, milk, any of various peptides, placenta, royal jelly, *Euglena* extract, hydrolyzable *Euglena* extract, *Euglena* oil, or any purified product or fermentation product of their components), a pH regulator (an inorganic acid, inorganic acid salt, organic acid or organic acid salt, or any of their derivatives), a vitamin (vitamin A group, vitamin B group, vitamin C, vitamin D group, ubiquinone or nicotinic acid amide, or any of their derivatives), a fermentate of yeast, *Aspergillus* or lactic acid bacteria, a galactomyces culture solution, a skin whitener (tranexamic acid, cetyl tranexamate hydrochloride, 4-n-butylresorcinol, arbutin, kojic acid, ellagic acid, licorice flavonoid, niacinamide or a vitamin C derivative), a ceramide or ceramide derivative, an anti-wrinkle agent (retinol or retinal or any of their derivatives, nicotinic acid amide, an oligopeptide, or any of their derivatives), a neutrophil elastase inhibitor, or a natural or synthetic component with an inhibiting effect on MMP-1 or MMP-2), titanium oxide, talc, mica, silica, zinc oxide, iron oxide, silicon, or their processed powders, in ranges that achieve the object of the pharmaceutical composition and cosmetic composition of this embodiment.

Components that may be added to the pharmaceutical composition and cosmetic composition of this embodiment are not limited to those mentioned above, and any components usable in pharmaceutical compositions and cosmetic compositions may be selected. When the pharmaceutical composition or cosmetic composition of this embodiment is to be used as a poultice, a base (such as kaolin or bentonite) or a gelling agent (such as a polyacrylic acid salt or polyvinyl alcohol) may be added in addition to the aforementioned components, in a range that achieves the object of the invention. When the pharmaceutical composition or cosmetic composition of this embodiment is to be used as a bath additive, a sulfate, hydrogencarbonate, borate, pigment or humectant may be added as appropriate in a range that achieves the object of the invention, and the composition may be prepared as a powder type or liquid drug type.

The pharmaceutical composition and cosmetic composition of this embodiment may be produced by a method that is commonly known in the technical field.

### Example 1

### (Example 1: Preparation of supernatant of culture medium used for maintenance culture of stem cells)

To 5 mL of L-glutamine (25030-081, Invitrogen), 5 mL of non-essential amino acid solution (11140-050, Invitrogen), 1 mL of 2-mercaptoethanol (21985-023, Invitrogen) and 2.5 mL of penicillin/streptomycin (15140-122, Invitrogen) there were added DMEM/F12 (10565-018, Invitrogen) and serum replacement (KSR: KnockOut Serum Replacement, registered trademark, 10828028, Invitrogen), to prepare culture medium at a total volume of 500 mL. To this culture medium there was added 0.2 mL of 10 µg/mL bFGF (basic fibroblast growth factor, R and D), for use as stem cell culture medium. The bFGF concentration of the stem cell culture medium was 4 ng/mL.

The stem cell culture medium prepared in this manner was used for adhesion maintenance culture of human iPS cells on feeder cells on an adhesion culture dish. The human iPS cells were subcultured once a week. During the subculturing, the human iPS cells were treated with stripping solution containing 0.25% trypsin, 0.1 mg/mL collagenase IV, 1 mmol/L CaCl₂ and 20% KSR.

The human iPS cells that had been maintenance cultured in this manner were detached from the adhesion culture dish using ES cell dissociating solution (TrypLE Select^{R}, ThermoFisher). The detached human iPS cells were seeded on a dish coated with laminin (Nippi, Inc.). Next, TeSR2 culture medium (Stem cell) with 10 µmοl/L ROCK inhibitor added was used for culturing of the human iPS cells for 1 week. Medium exchange was carried out every day.

The culture medium was then exchanged with stem cell culture medium, and the supernatant of the stem cell culture medium was collected after 2 days. After centrifuging the collected stem cell culture medium supernatant at 1500 rpm for 5 minutes and again collecting the culture medium supernatant, it was centrifuged at 3000 rpm for 3 minutes and the supernatant of the centrifuged stem cell culture medium was filtered with a 0.22 µm filter. The supernatant of the filtered stem cell culture medium was used as a supernatant solution for Example 1.

The maintenance cultured iPS cells were confirmed to be positive for the undifferentiated markers NANOG, OCT3/4 and TRA 1-60.

### (Example 2: Preparation of supernatant of culture medium used for maintenance culture of stem cells)

Human iPS cells were maintenance cultured in the same manner as Example 1. The human iPS cells were then detached from the adhesion culture dish in the same manner as Example 1 and divided into single cells. Next, the human iPS cells were seeded in stem cell culture medium that had been gelled by addition of gellan gum and 10 µmοl/L of ROCK inhibitor (Selleck), and the human iPS cells were suspension cultured for 21 days. The gelled stem cell culture medium was replenished in the culturing vessel once every two days during the period.

The gelled stem cell culture medium in which the human iPS cells were suspended was then filtered with a mesh filter to remove the cell masses. The filtered gelled stem cell culture medium was also centrifuged at 1500 rpm for 5 minutes to precipitate the cells and gel, and after again collecting the supernatant of the centrifuged stem cell culture medium, it was centrifuged at 3000 rpm for 3 minutes, and the supernatant of the centrifuged stem cell culture medium was filtered with a 0.22 µm filter. The supernatant of the filtered stem cell culture medium was used as a supernatant solution for Example 2.

The maintenance cultured iPS cells were confirmed to be positive for the undifferentiated markers NANOG, OCT3/4 and TRA 1-60.

### (Example 3: Preparation of supernatant of culture medium used for maintenance culture of stem cells)

Human iPS cells were maintenance cultured in the same manner as Example 1. The human iPS cells were then detached from the adhesion culture dish in the same manner as Example 1 and divided into single cells. Next, the human iPS cells were seeded in stem cell culture medium that had been gelled by addition of gellan gum and 100 µmοl/L of ROCK inhibitor (Selleck), and the human iPS cells were suspension cultured for 14 days. The gelled stem cell culture medium was replenished in the culturing vessel once every two days during the period.

The gelled stem cell culture medium in which the human iPS cells were suspended was then filtered with a mesh filter to remove the cell masses. The filtered gelled stem cell culture medium was also centrifuged at 1500 rpm to precipitate the cells and gel, and after again collecting the supernatant of the centrifuged stem cell culture medium, it was centrifuged at 3000 rpm for 3 minutes, and the supernatant of the centrifuged stem cell culture medium was filtered with a 0.22 µm filter. The supernatant of the filtered stem cell culture medium was used as a supernatant solution for Example 3.

The maintenance cultured iPS cells were confirmed to be positive for the undifferentiated markers NANOG, OCT3/4 and TRA 1-60.

### (Comparative Example: Preparation of supernatant of culture medium obtained by culturing differentiated cells)

Human iPS cells were cultured following the Examples described in Japanese Unexamined Patent Publication No. 2016-128396. Specifically, the same stem cell culture medium as in Example 1 was used for adhesion maintenance culture of human iPS cells on feeder cells on an adhesion culture dish. The human iPS cells were subcultured once a week. During the subculturing, the human iPS cells were treated with stripping solution containing 0.25% trypsin, 0.1 mg/mL collagenase IV, 1 mmol/L CaCl₂ and 20% KSR.

The human iPS cells that had been cultured in this manner were detached from the adhesion culture dish using ES cell dissociating solution (TrypLE Select^{R}, ThermoFisher). The detached human iPS cells were suspension cultured for 1 week among non-gelled human iPS cells placed in a non-adhesion culture dish. This resulted in formation of an embryo body (EB). The formed embryo body was seeded on an adhesion culture dish and grown for 1 week in 10% FBS-containing DMEM (outgrowth).

The cells were then detached from the adhesion culture dish using a 0.05% trypsin-EDTA solution and divided into single cells, which were seeded onto a fresh adhesion culture dish. The cells were subsequently cultured for one week using 10% FBS-containing DMEM as the culture medium.

After confirming 70% to 80% confluence of the cells, the culture medium was exchanged with serum-free medium (FBS-free DMEM) and culturing was carried out for 2 days, after which the culture medium supernatant was collected. After centrifuging the collected stem cell culture medium supernatant at 1500 rpm for 5 minutes and again collecting the culture medium supernatant, it was centrifuged at 3000 rpm for 3 minutes and the culture medium supernatant was again collected and used as the supernatant solution for the Comparative Example.

The cultured cells were negative for the undifferentiated markers NANOG, OCT3/4 and TRA 1-60, and were therefore confirmed to be differentiated cells.

### (Example 4: Fibroblast proliferation test)

DMEM culture medium with addition of 10% FBS and 1% penicillin-streptomycin was prepared as growth medium A. Adult normal human fibroblasts (KF-4109, Strain No.01035, Kurabo Industries, Ltd.) were then suspended with growth medium A to a concentration of 5 × 10³ cells/0.1 mL/well, seeded in a 96-well plate, and cultured for 1 day in a CO₂ incubator (5% CO₂, 37°C).

DMEM culture medium with addition of 1% FBS and 1% penicillin-streptomycin was prepared as test medium A. Next, the supernatant solutions of Examples 1 to 3 and the Comparative Example were each mixed with test medium A in a volume ratio of 10.00:90.00, to obtain supernatant-added culture medium A for Examples 1 to 3 and the Comparative Example, at a concentration of 10.00 v/v%. The growth medium A in some of the wells was exchanged with the supernatant-added culture medium A of Examples 1 to 3 and the Comparative Example.

As a negative control, the growth medium A in some of the wells was exchanged with DMEM culture medium without addition of 1% FBS and 1% penicillin-streptomycin (non-added test medium A). As another negative control, the growth medium A in some of the wells was exchanged with diluted test medium A obtained by mixing DMEM/F12 with test medium A in a volume ratio of 10.00:90.00.

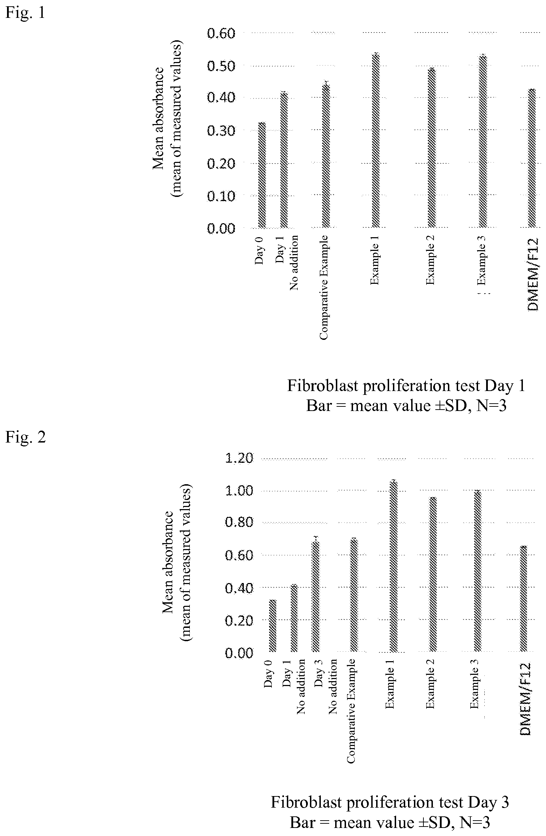

The fibroblasts were cultured in the exchanged medium for 1 day and 3 days, and the viable cell count was measured by the WST-8 method, using viable cell count-measuring reagent SF (Cat. No. 07553-15, Nacalai Tesque, Inc.) and a plate reader (Varioskan MicroPlate Reader, Thermo Scientific). The results are shown in Fig. 1 and Fig. 2. It was confirmed that growth of fibroblasts was superior when using the supernatant-added culture medium A of Examples 1 to 3 with a concentration of 10.00 v/v%, compared to using the non-added test medium A, the diluted test medium A or the supernatant-added culture medium A of the Comparative Example. The results also suggested that a supernatant of stem cell culture medium is effective for increasing cell viability. The cells become subjected to physical stress such as pressure and chemical stress by the release agent during detachment from the dish during subculturing and when being divided into single cells. However, since the stressed cells proliferated by the supernatant-added culture medium in the Examples, this suggested that the supernatant-added culture medium of the Examples alleviated the stress experienced by the cells and protected the cells from stress, thus stabilizing the cells and increasing their viability. The results suggested that the supernatant-added culture medium of the Examples protects the nucleic acids, proteins, protein complexes, lipoproteins, ribosomes and biological membranes present in the cells. The biological membranes referred to here include cell membranes.

### (Example 5: Type I collagen and hyaluronic acid production test using fibroblasts)

Adult normal human fibroblasts were cultured for 1 day with growth medium A, in the same manner as Example 4. The growth medium A in some of the wells was exchanged with the supernatant-added culture medium A of Examples 1 to 3 or the Comparative Example, in the same manner as Example 4, except that the concentration was 1.00 v/v%, 10.00 v/v% or 100.0 v/v%.

The growth medium A in some of the wells was not exchanged, as a positive control. As a negative control, the growth medium A in some of the wells was exchanged with non-added test medium A. As another negative control, the growth medium A in some of the wells was exchanged with diluted test medium A prepared in the same manner as Example 4.

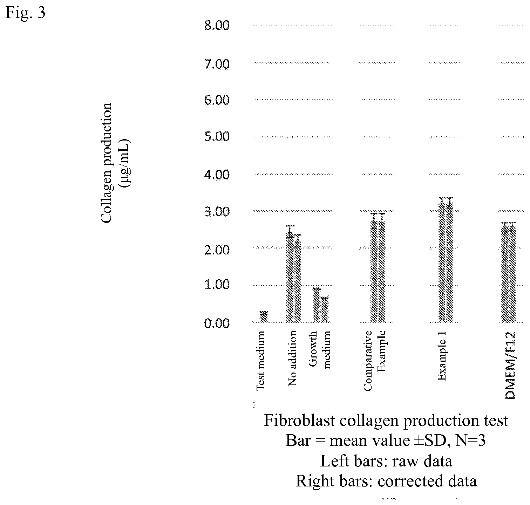

After exchanging the culture medium, the fibroblasts were cultured for 3 days and the culture medium supernatant was collected and stored at -80°C. The culture medium supernatant was then thawed and the type I collagen concentration of the culture medium supernatant was measured using a human collagen type 1 ELISA kit (Cat. No. EC1-E105). The hyaluronic acid concentration of the culture medium supernatant was measured using DueSet Hyaluronan (Cat. No. DY3614, R&D Systems). The results are shown in Fig. 3, Fig. 4 and Fig. 5.

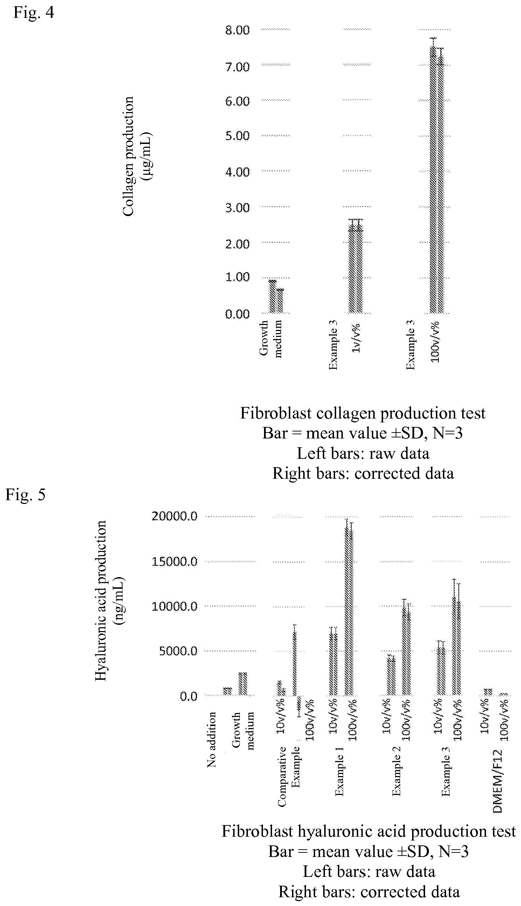

In Fig. 3 and Fig. 4, the right corrected bars represent corrected data, obtained by subtracting the amount of collagen originally present in the culture medium before culturing the fibroblasts. The left raw data bars represent the data before correction. As shown in Fig. 3, the amount of type I collagen production increased when the supernatant-added culture medium A of Example 1 at 1.0 v/v% concentration was used, compared to when the non-added test medium A, the growth medium A, the diluted test medium A or the supernatant-added culture medium A of the Comparative Example was used. As shown in Fig. 4, the amount of type I collagen production increased markedly when the supernatant-added culture medium A of Example 3 at 1.0 v/v% and 100.0 v/v% concentrations was used, compared to when the growth medium A was used. The results therefore suggested that a supernatant of a stem cell culture medium is effective for promoting collagen production and preventing formation of or ameliorating skin wrinkles and sagging.

In Fig. 5, the right corrected bars represent corrected data, obtained by subtracting the amount of hyaluronic acid originally present in the culture medium before culturing the fibroblasts. The left raw data bars represent the data before correction. The amount of hyaluronic acid produced markedly increased when the supernatant-added culture medium A of Examples 1 to 3 at 10.0 v/v% and 100.0 v/v% concentrations was used, compared to when the non-added test medium A, the diluted test medium A or the supernatant-added culture medium A of the Comparative Example was used. The results therefore suggested that a supernatant of a stem cell culture medium is effective for promoting hyaluronic acid production and preventing formation of or ameliorating skin wrinkles and sagging resulting from reduced hyaluronic acid.

### (Example 6: Epidermal cell migration test)

As growth medium B, there was prepared 500 mL of epidermal cell culture medium (HuMedia-KG2, Kurabo Industries, Ltd.) containing a growth additive (10 µg/mL insulin, 0.1 ng/mL hEGF, 0.67 µg/mL hydrocortisone, 4 µL/mL bovine pituitary extract BPE) and an antimicrobial agent (50 µg/mL gentamycin, 50 ng/mL amphotericin).

The adult normal human epidermal cells were treated for 2 hours with 10 µg/mL Mitomycin C (Cat. No. 20898-21, Nacalai Tesque) to halt the cell division. The human epidermal cells were then suspended with growth medium B to a concentration of 4 × 10⁴ cells/0.1 mL/well and seeded on a collagen-coated plate of a kit for measurement of cell migration ability (Oris Cell Migration Assay^{R}), and they were cultured for 1 day in a CO₂ incubator (5% CO₂, 37°C), whereby the epidermal cells became anchored to the outer edge section of the plate stopper, which was not blocked by the stopper. The stopper was then removed from the plate.

As test medium B, there was prepared culture medium obtained by adding 500 mL of an antimicrobial agent (50 µg/mL gentamycin and 50 nm/mL amphotericin) to epidermal cell culture medium. Next, the supernatant solutions of Examples 1 to 3 and the Comparative Example were each mixed with test medium B in a volume ratio of 10.0:90.0 and 1.0:99.0, respectively, to obtain supernatant-added culture medium B for Examples 1 to 3 and the Comparative Example. Some of the growth medium B on the plate was exchanged with the supernatant-added culture medium B of Examples 1 to 3 and the Comparative Example, respectively.

Some of the growth medium B on the plate was also exchanged with epidermal cell culture medium without addition of growth additive (non-added test medium B), as a negative control. As another negative control, some of the growth medium B on the plate was exchanged with diluted test medium B obtained by mixing DMEM/F12 with test medium B in a volume ratio of 10.0:90.0 or 1.0:99.0.

During the course of wound healing, epidermal cells migrate toward the wound, resulting in constriction of the wound. For this Example, a plate reader was used to analyze whether or not epidermal cells had migrated to the region blocked by the stopper. Specifically, the epidermal cells were stained with live cell staining reagent (Calcein AM, Cat. NO.341-07901, DOJINDO) 23 hours after exchange of the culture medium, and a plate reader (Varioskan MicroPlate Reader, Thermo Scientific) was used to measure the fluorescence with a wavelength of 538 nm with respect to excitation light with a wavelength of 485 nm.

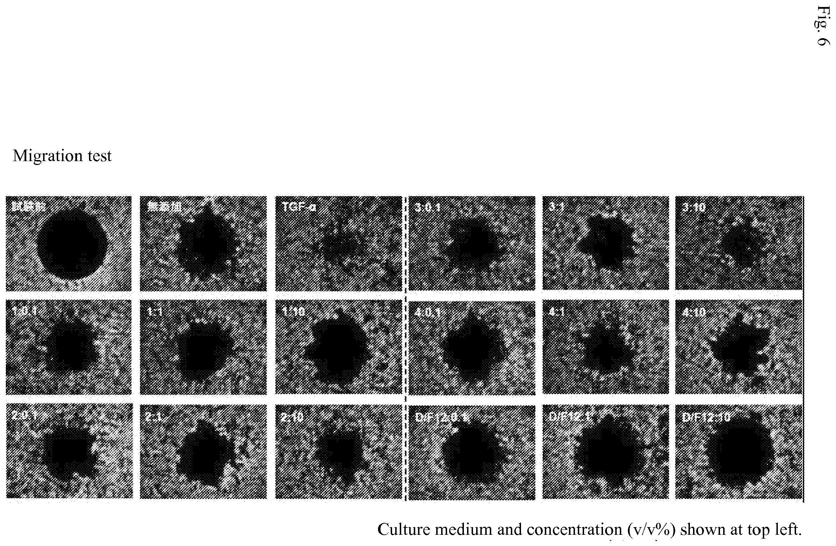

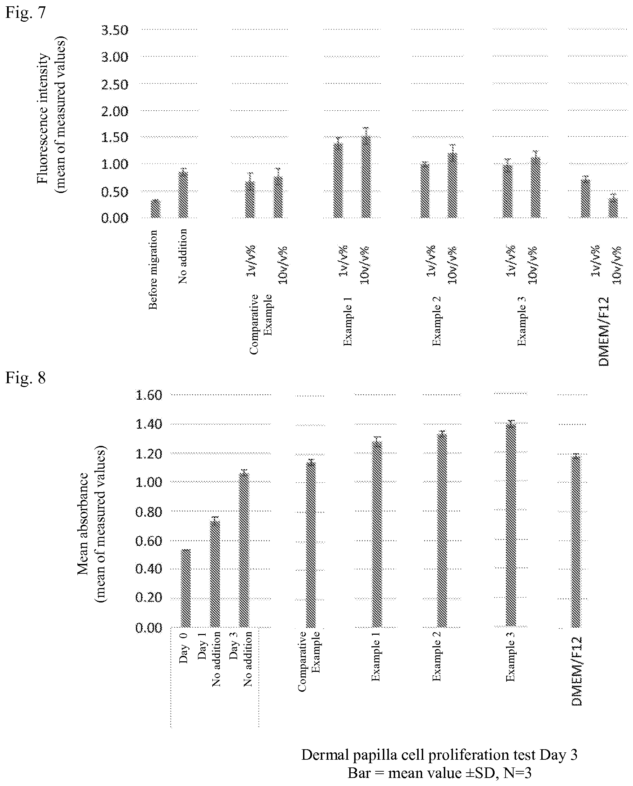

The results are shown in Fig. 6 and Fig. 7. A significant accelerating effect on migration ability of epidermal cells was found when the supernatant-added culture medium B of Examples 1 to 3 was used at concentrations of 1.0 v/v% and 10.0 v/v%, compared to the non-added test medium B, the diluted test medium B, and the supernatant-added culture medium B of the Comparative Example. The results therefore indicated that a supernatant of stem cell culture medium is effective for wound healing. The results also suggested that a supernatant of stem cell culture medium is effective for preventing formation of or ameliorating skin spots and color irregularities in the skin surface layer that are produced when damaged skin is exposed to ultraviolet light. Furthermore, the results suggested that a supernatant of stem cell culture medium is effective for stabilizing and increasing the viability of cells. The cells become subjected to physical stress such as pressure and chemical stress by the release agent during detachment from the dish during subculturing and when being divided into single cells. However, since the stressed cells proliferated by the supernatant-added culture medium in the Examples, this suggested that the supernatant-added culture medium of the Examples alleviated the stress experienced by the cells and protected the cells from stress, thus stabilizing the cells and increasing their viability. These results suggested that the supernatant-added culture medium of the Examples protects the nucleic acids, proteins, protein complexes, lipoproteins, ribosomes and biological membranes present in the cells. The biological membranes referred to here include cell membranes.

### (Example 7: Dermal papilla cell proliferation test)

As growth medium C, there was prepared a dermal papilla cell special medium (Cat. No. TMTPGM-250, TOYOBO), with addition of a special-purpose additive (fetal calf serum, insulin/transferrin/triiodothyronine mixture, bovine pituitary extract, cyproterone acetate). Next, normal human dermal papilla cells (Cat. No. CA60205a, Lot. No. 2868, TOYOBO) were suspended with growth medium C to a concentration of 1.2 × 10⁴ cells/0.3 mL/well, seeded in a type I collagen-coated 48-well plate, and cultured for 1 day in a CO₂ incubator (5% CO₂, 37°C).

The supernatant solutions of Examples 1 to 3 and the Comparative Example were each mixed with dermal papilla cell special medium that lacked additives (non-added test medium C) at a volume ratio of 30.0:70.0, to obtain supernatant-added culture medium C for Examples 1 to 3 and the Comparative Example. The growth medium C in some of the wells was exchanged with the supernatant-added culture medium C of Examples 1 to 3 and the Comparative Example.

The growth medium C in some of the wells was also exchanged with dermal papilla cell special medium that lacked additives (non-added test medium C), as a negative control. As another negative control, the growth medium C in some of the wells was exchanged with diluted test medium C obtained by mixing DMEM/F12 with non-added test medium C in a volume ratio of 30.0:70.0.

The dermal papilla cell were cultured for 3 days in the exchanged culture medium, and the viable cell count was measured by the WST-8 method. The results are shown in Fig. 8. It was confirmed that growth of dermal papilla cells was superior when using the supernatant-added culture medium C of Examples 1 to 3 with a concentration of 30.0 v/v%, compared to using the non-added test medium C or the diluted test medium C. The results therefore suggested that a supernatant of stem cell culture medium has a hair-growing and hair-generating effect, which includes treating thin hair, preventing alopecia, accelerating hair formation and accelerating hair growth.

### (Example 8: FGF-7 and VEGF-production test using dermal papilla cells)

Normal human dermal papilla cells were cultured for 1 day with growth medium C, in the same manner as Example 7. The growth medium C in some of the wells was then exchanged with the supernatant-added culture medium C of Examples 1 to 3 or the Comparative Example, in the same manner as Example 7, except that the concentration was 0.3 v/v%, 30.0 v/v% or 100.0 v/v%.

As a negative control, the growth medium C in some of the wells was exchanged with non-added test medium C or diluted test medium C. As a reference control, the growth medium C in some of the wells was exchanged with adenosine-added medium obtained by adding 100 µmol/L of adenosine to dermal papilla cell special medium, or adenosine-added medium obtained by adding 30 µmol/L of minoxidil to dermal papilla cell special medium. In addition, as a minoxidil vehicle control, the growth medium C in some of the wells was exchanged with DMSO-added medium obtained by adding 0.1% DMSO to dermal papilla cell special medium.

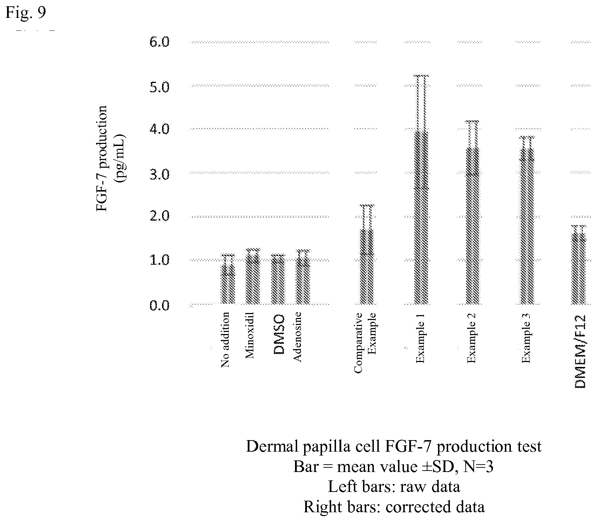

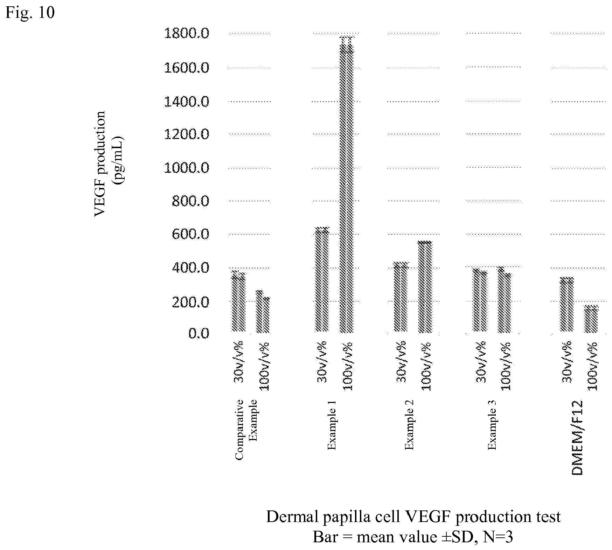

After exchanging the culture medium, the dermal papilla cells were cultured for 3 days and the culture medium supernatant was collected and stored at -80°C. The culture medium supernatant was then thawed and the fibroblast growth factor 7 (FGF-7) concentration in the culture medium supernatant was measured using an FGF-7 Human ELISA kit (Cat. No. ab100519, Abcam). The vascular endothelial growth factor (VEGF) concentration of the culture medium supernatant was also measured using a Human VEGF Quantikine ELISA (Cat. No. DVE00, R&D Systems). The results are shown in Fig. 9 and Fig. 10.

In Fig. 9, the right corrected bars represent corrected data, obtained by subtracting the amount of FGF-7 originally present in the culture medium before culturing the dermal papilla cells. The left raw data bars represent the data before correction. It was confirmed that FGF-7 production was significantly increased when using the supernatant-added culture medium C of Examples 1 to 3 with a concentration of 0.3 v/v%, compared to using the non-added test medium C, the diluted test medium C or the supernatant-added culture medium C of the Comparative Example.

In Fig. 10, the right corrected bars represent corrected data, obtained by subtracting the amount of VEGF originally present in the culture medium before culturing the dermal papilla cells. The left raw data bars represent the data before correction. It was confirmed that VEGF production was significantly increased when using the supernatant-added culture medium C of Examples 1 to 3 with a concentration of 30.0 v/v% or 100.0 v/v%, compared to using the diluted test medium C or the supernatant-added culture medium C of the Comparative Example.

## Claims

1. A pharmaceutical composition or a pharmaceutical composition starting material that includes a supernatant of a culture medium that has been used for maintenance culture of stem cells in an undifferentiated state.

2. The pharmaceutical composition or pharmaceutical composition starting material according to claim 1, wherein the stem cells are pluripotent stem cells.

3. An agent that prevents formation of or ameliorates skin blemishes, wrinkles or sagging, which comprises the pharmaceutical composition or pharmaceutical composition starting material according to claim 1 or 2.

4. A cosmetic composition or cosmetic composition starting material that includes a supernatant of a culture medium that has been used for maintenance culture of stem cells in an undifferentiated state.

5. The cosmetic composition or cosmetic composition starting material according to claim 4, wherein the stem cells are pluripotent stem cells.

6. An agent that prevents formation of or ameliorates skin blemishes, wrinkles or sagging, which comprises the cosmetic composition or cosmetic composition starting material according to claim 4 or 5.

7. A collagen production promoter or a collagen production promoter starting material, that includes a supernatant of a culture medium that has been used for maintenance culture of stem cells in an undifferentiated state.

8. The collagen production promoter or collagen production promoter starting material according to claim 7, wherein the stem cells are pluripotent stem cells.

9. A hyaluronic acid production promoter or a hyaluronic acid production promoter starting material, that includes a supernatant of a culture medium that has been used for maintenance culture of stem cells in an undifferentiated state.

10. The hyaluronic acid production promoter or hyaluronic acid production promoter starting material according to claim 9, wherein the stem cells are pluripotent stem cells.

11. A wound treatment agent or a wound treatment agent starting material that includes a supernatant of a culture medium that has been used for maintenance culture of stem cells in an undifferentiated state.

12. The wound treatment agent or wound treatment agent starting material according to claim 11, wherein the stem cells are pluripotent stem cells.

13. An epidermal cell growth promoter or an epidermal cell growth promoter starting material that includes a supernatant of a culture medium that has been used for maintenance culture of stem cells in an undifferentiated state.

14. The epidermal cell growth promoter or epidermal cell growth promoter starting material according to claim 13, wherein the stem cells are pluripotent stem cells.

15. A hair growth agent or hair growth agent starting material that includes a supernatant of a culture medium that has been used for maintenance culture of stem cells in an undifferentiated state.

16. The hair growth agent or hair growth agent starting material according to claim 15, wherein the stem cells are pluripotent stem cells.

17. A hair restorer or hair restorer starting material that includes a supernatant of a culture medium that has been used for maintenance culture of stem cells in an undifferentiated state.

18. The hair restorer or hair restorer starting material according to claim 17, wherein the stem cells are pluripotent stem cells.

19. A dermal papilla cell activating agent or a dermal papilla cell activating agent starting material that includes a supernatant of a culture medium that has been used for maintenance culture of stem cells in an undifferentiated state.

20. The dermal papilla cell activating agent or dermal papilla cell activating agent starting material according to claim 19, wherein the stem cells are pluripotent stem cells.

21. A fibroblast growth factor family production promoter or a fibroblast growth factor family production promoter starting material that includes a supernatant of a culture medium that has been used for maintenance culture of stem cells in an undifferentiated state.

22. The fibroblast growth factor family production promoter or fibroblast growth factor family production promoter starting material according to claim 21, wherein the stem cells are pluripotent stem cells.

23. A vascular endothelial growth factor production promoter or a vascular endothelial growth factor production promoter starting material that includes a supernatant of a culture medium that has been used for maintenance culture of stem cells in an undifferentiated state.

24. The vascular endothelial growth factor production promoter or vascular endothelial growth factor production promoter starting material according to claim 23, wherein the stem cells are pluripotent stem cells.

25. A cytoprotective agent or cytoprotective agent starting material which protects cells from stress, and that includes a supernatant of a culture medium that has been used for maintenance culture of stem cells in an undifferentiated state.

26. The cytoprotective agent or cytoprotective agent starting material according to claim 25, wherein the stem cells are pluripotent stem cells.

27. A cell viability improver or a cell viability improver starting material which increases the viability of stressed cells, and that includes a supernatant of a culture medium that has been used for maintenance culture of stem cells in an undifferentiated state.

28. The cell viability improver or cell viability improver starting material according to claim 27, wherein the stem cells are pluripotent stem cells.

29. A biological substance protective agent or a biological substance protective agent starting material, which protects one or more biological substances selected from the group consisting of nucleic acids, proteins, protein complexes, lipoproteins, ribosomes and biological membranes from stress, and that includes a supernatant of a culture medium that has been used for maintenance culture of stem cells in an undifferentiated state.

30. The biological substance protective agent or biological substance protective agent starting material according to claim 29, wherein the stem cells are pluripotent stem cells.
